# EUROPEAN PATENT SPECIFICATION

(11) **EP 1 356 119 B1**
(45) Date of publication and mention of the grant of the patent: **26.09.2007**
(21) Application number: 02712025.2
(22) Date of filing: 30.01.2002
(51) Int. Cl.: C12Q 1/68

(54) **THE PREPARATION OF POLYNUCLEOTIDE ARRAYS**
HERSTELLUNG VON MATRIZEN AUS POLYNUKLEOTIDEN
PREPARATION D'UN RESEAU DE POLYNUCLEOTIDES

(30) Priority: 30.01.2001 US 771708
(43) Date of publication of application: 29.10.2003
(60) Divisional application: 07008271.4
(73) Proprietor: Solexa Ltd., Saffron Walden, Essex CB10 1XL (GB)
(72) Inventor: BARNES, Colin, Solexa Ltd., Saffron Walden, Essex CB10 1XL (GB); BALASUBRAMANIAN, Shankar, University of Cambridge, Cambridge CB2 1EW (GB); KLENERMAN, David, University of Cambridge, Cambridge CB2 1EW (GB); OSBORNE, Mark, Allen, Brighton BN1 3JB (GB)
(74) Representative: Murphy, Colm Damien
(86) International application number: PCT/GB2002/000438
(87) International publication number: WO 2002/061126

(56) References cited:
- EP-A- 1 077 264
- WO-A-00/06770
- WO-A-98/44151
- US-A- 5 314 829
- DATABASE WPI Section Ch, Week 199840 Derwent Publications Ltd., London, GB; Class B04, AN 1998-465919 XP002219825 & SE 9 500 589 A (R. RIGLER ), 18 August 1996 (1996-08-18)
- AILEEN CONSTANS: "To Dream the Not-So-Impossible Genomics Dream" THE SCIENTIST, vol. 16, no. 20, 14 October 2002 (2002-10-14), page 53 XP002219824 Philadelphia, PA, US

## Description

### Field of the Invention

This invention relates to fabricated arrays of polynucleotides, and to their analytical applications.

### Background of the Invention

Advances in the study of molecules have been led, in part, by improvement in technologies used to characterise the molecules or their biological reactions. In particular, the study of nucleic acids, DNA and RNA, has benefited from developing technologies used for sequence analysis and the study of hybridisation events.

An example of the technologies that have improved the study of nucleic acids, is the development of fabricated arrays of immobilised nucleic acids. These arrays typically consist of a high-density matrix of polynucleotides immobilised onto a solid support material. Fodor et al., Trends in Biotechnology (1994) 12:19-26, describes ways of assembling the nucleic acid arrays using a chemically sensitised glass surface protected by a mask, but exposed at defined areas to allow attachment of suitably modified nucleotides. Typically, these arrays may be described as "many molecule" arrays, as distinct regions are formed on the solid support comprising a high density of one specific type of polynucleotide.

An alternative approach is described by Schena et al., Science (1995) 270:467-470, where samples of DNA are positioned at predetermined sites on a glass microscope slide by robotic micropipetting techniques. The DNA is attached to the glass surface along its entire length by non-covalent electrostatic interactions. However, although hybridisation with complementary DNA sequences can occur, this approach may not permit the DNA to be freely available for interacting with other components such as polymerase enzymes, DNA-binding proteins etc.

WO-A-96/27025 is a general disclosure of single molecule arrays. Although sequencing procedures are disclosed, there is little description of the applications to which the arrays can be applied. There is also only a general discussion on how to prepare the arrays.

### Summary of the Invention

According to the present invention, a device comprises a high density array of relatively short molecules and relatively long polynucleotides immobilised on the surface of a solid support, wherein the polynucleotides are at a density that permits individual resolution of those parts that extend beyond the relatively short molecules. In this aspect, the shorter molecules help to control the density ofthe polynucleotides, providing a more uniform array of single polynucleotide molecules, thereby improving imaging. The small molecules may also prevent non-specific binding of reagents to the solid support, and therefore reduce background interference. For example, in the context of a polymerase reaction to incorporate nucleoside triphosphates onto a strand complementary to a long polynucleotide, the small molecules prevent the polymerase and nucleosides from attaching to the solid support surface, which may otherwise interfere with the imaging process.

The shorter molecules may also ensure that each polynucleotide is maintained upright, preventing the polynucleotides from interacting lengthwise with the solid support, which may otherwise prevent efficient interaction with a reagent, e.g. a polymerase. This may also prevent the fluorophore being quenched by the surface and therefore lead to more accurate imaging of the single polynucleotides.

According to a second aspect of the invention, a method for the production of an array of polynucleotides which are at a density that permits individual resolution, comprises arraying on the surface of a solid support, a mixture of relatively short molecules and relatively long polynucleotides, wherein the short molecules are arrayed in an amount in excess of the polynucleotides.

The arrays of the present invention comprise what are effectively single analysable polynucleotides. This has many important benefits for the study ofthe polynucleotides and their interaction with other biological molecules. In particular, fluorescence events occurring on each polynucleotide can be detected using an optical microscope linked to a sensitive detector, resulting in a distinct signal for each polynucleotide.

When used in a multi-step analysis of a population of single polynucleotides, the phasing problems that are encountered using high density (multi-molecule) arrays of the prior art, can be reduced or removed. Therefore, the arrays also permit a massively parallel approach to monitoring fluorescent or other events on the polynucleotides. Such massively parallel data acquisition makes the arrays extremely useful in a wide range of analysis procedures which involve the screening/characterising of heterogeneous mixtures of polynucleotides.

The preparation ofthe arrays requires only small amounts of polynucleotide sample and other reagents, and can be carried out by simple means.

### Description of the Drawings

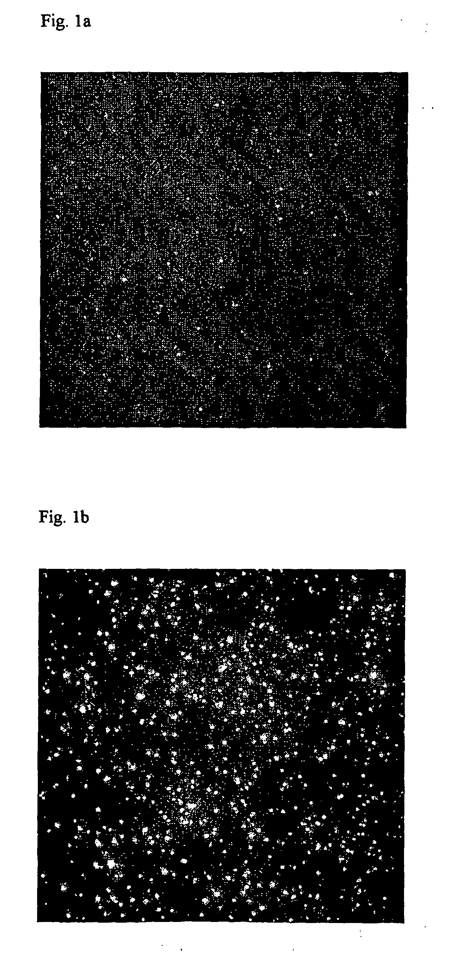

Figures 1a and b are images of a single polynucleotide array, where single polynucleotides are indicated by the detection of a fluorescent signal generated on the array.

### Description of the Invention

The single polynucleotide array devices of the present invention are fabricated to include a "monolayer" of relatively short molecules that coat the surface of a solid support material and provide a flexible means to control the density of the single polynucleotides and optionally to prevent non-specific binding of reagents to the solid support.

In the context of the present invention, the terms "relatively short" and "relatively long" should be interpreted to mean that the "relatively long" polynucleotides extend above the "relatively short" molecules when arrayed.

The single polynucleotides immobilised onto the surface of a solid support should be capable of being resolved by optical means. This means that, within the resolvable area of the particular imaging device used, there must be one or more distinct images each representing one polynucleotide. Typically, the polynucleotides of the array are resolved using a single molecule fluorescence microscope equipped with a sensitive detector, e.g. a charge-coupled device (CCD). Each polynucleotide of the array may be imaged simultaneously or, by scanning the array, a fast sequential analysis can be performed.

The polynucleotides of the array are typically DNA or RNA, although nucleic acid mimics, e.g. PNA or 2'-O-Meth-RNA, are within the scope of the invention. The polynucleotides are formed on the array to allow interaction with other molecules. It is therefore important to immobilise the polynucleotides so that the portion of the polynucleotide not physically attached to solid support is capable of being interrogated. In some applications all the polynucleotides in the single array will be the same, and may be used to capture molecules that are largely distinct. In other applications, the polynucleotides on the array may all, or substantially all, be different, e.g. less than 50%, preferably less than 30% of the polynucleotides will be the same.

The term "single molecule" is used herein to distinguish from high density multi-molecule (polynucleotide) arrays in the prior art, which may comprise distinct clusters of many polynucleotides of the same type.

The term "individually resolved" is used herein to indicate that, when visualised, it is possible to distinguish one polynucleotide on the array from its neighbouring polynucleotides. Visualisation may be effected by the use of reporter labels, e.g. fluorophores, the signal of which is individually resolved. There may be some polynucleotides present on the solid support that are not capable of being individually resolved, however, these can be discounted during imaging, provided that the majority of the polynucleotides can be resolved at the single molecule level.

The term "interrogate" is used herein to refer to any interaction of the arrayed polynucleotide with any other molecule, e.g. with a polymerase or nucleoside triphosphate.

The density ofthe arrays is not critical. However, the present invention can make use of a high density of single polynucleotides, and these are preferable. For example, arrays with a density of 10⁶-10⁹ polynucleotides per cm² may be used. Preferably, the density is at least 10⁷/cm² and typically up to 10⁸/cm². These high density arrays are in contrast to other arrays which may be described in the art as "high density" but which are not necessarily as high and/or which do not allow single molecule resolution.

The shorter molecules will typically be present on the array at much higher density, to coat the remaining surface of the solid support. The shorter molecules may therefore be brought into contact with the solid support at an excess concentration. Preferably, the small molecules are at a density of from 10⁸ to 10¹⁴ molecules/cm², more preferably greater than 10¹² molecules/cm².

Using the methods and apparatus of the present invention, it may be possible to image at least 10⁵ or 10⁸ polynucleotides/cm², preferably at least 10⁷ polynucleotides/cm². Fast sequential imaging may be achieved using a scanning apparatus; shifting and transfer between images may allow higher numbers of polynucleotides to be imaged.

The extent of separation between the individual polynucleotides on the array will be determined, in part, by the particular technique used to resolve the individual polynucleotide. Apparatus used to image molecular arrays are known to those skilled in the art. For example, a confocal scanning microscope may be used to scan the surface of the array with a laser to image directly a fluorophore incorporated on the individual polynucleotide by fluorescence. Alternatively, a sensitive 2-D detector, such as a charge-coupled device, can be used to provide a 2-D image representing the individual polynucleotides on the array.

Resolving single polynucleotides on the array with a 2-D detector can be done if, at 100 x magnification, adjacent polynucleotides are separated by a distance of approximately at least 250nm, preferably at least 300nm and more preferably at least 350nm. It will be appreciated that these distances are dependent on magnification, and that other values can be determined accordingly, by one of ordinary skill in the art.

Other techniques such as scanning near-field optical microscopy (SNOM) are available which are capable of greater optical resolution, thereby permitting more dense arrays to be used. For example, using SNOM, adjacent polynucleotides may be separated by a distance of less than 100nm, e.g. 10nm. For a description of scanning near-field optical microscopy, see Moyer et al., Laser Focus World (1993) 29(10).

An additional technique that may be used is surface-specific total internal reflection fluorescence microscopy (TIRFM); see, for example, Vale et al., Nature, (1996) 380: 451-453). Using this technique, it is possible to achieve wide-field imaging (up to 100 µm x 100 µm) with single molecule sensitivity. This may allow arrays of greater than 10⁷ resolvable polynucleotides per cm² to be used.

Additionally, the techniques of scanning tunnelling microscopy (Binnig et al., Helvetica Physica Acta (1982) 55:726-735) and atomic force microscopy (Hansma et al., Ann. Rev. Biophys. Biomol. Struct. (1994) 23:115-139) are suitable for imaging the arrays of the present invention. Other devices which do not rely on microscopy may also be used, provided that they are capable of imaging within discrete areas on a solid support.

The devices according to the invention comprise immobilised polynucleotides and other immobilised molecules. The other molecules are relatively short compared to the polynucleotides and are used to control the density of the polynucleotides. They may also prevent non-specific attachment of reagents, e.g. nucleoside triphosphates, with the solid support, thereby reducing background interference. In one embodiment, the shorter molecules are also polynucleotides. However, many different molecules may be used, e.g. peptides, proteins, polymers and synthetic chemicals, as will be apparent to the skilled person. The preferred molecules are organic molecules that contain groups that can react with the surface of a solid support.

Preparation of the devices may be carried out by first preparing a mixture of the relatively long polynucleotides and of the relatively short molecules. Usually, the concentration of the latter will be in excess of that of the long polynucleotides. The mixture is then placed in contact with a suitably prepared solid support, to allow immobilisation to occur.

Single polynucleotides may be immobilised to the surface of a solid support by any known technique, provided that suitable conditions are used to ensure adequate separation. Density of the polynucleotide molecules may be controlled by dilution. The gaps between the polynucleotides can be filled in with short molecules (capping groups) that may be small organic molecules or may be polynucleotides of different composition. The formation of the array of individually resolvable "longer" polynucleotides permits interrogation of those polynucleotides that are different from the bulk of the molecules.

Suitable solid supports are available commercially, and will be apparent to the skilled person. The supports may be manufactured from materials such as glass, ceramics, silica and silicon. Supports with a gold surface may also be used. The supports usually comprise a flat (planar) surface, or at least a structure in which the polynucleotides to be interrogated are in the same plane. Any suitable size may be used. For example, the supports might be of the order of 1-10 cm in each direction.

Immobilisation may be by specific covalent or non-covalent interactions. Covalent attachment is preferred. Immobilisation of a polynucleotide will be carried out at either the 5' or 3' position, so that the polynucleotide is attached to the solid support at one end only. However, the polynucleotide may be attached to the solid support at any position along its length, the attachment acting to tether the polynucleotide to the solid support; this is shown for the hairpin constructs, described below. The immobilised (relatively long) polynucleotide is then able to undergo interactions with other molecules or cognates at positions distant from the solid support. Immobilisation in this manner results in well separated long polynucleotides. The advantage of this is that it prevents interaction between neighbouring long polynucleotides on the array, which may hinder interrogation of the array.

Suitable methods for forming the devices with relatively short molecules and relatively long polynucleotides will be apparent to the skilled person, based on conventional chemistries. The aim is to produce a highly dense layer ofthe relatively short molecules, interspersed with the relatively large polynucleotides which are at a density that permits resolution of each single polynucleotide.

A first step in the fabrication of the arrays will usually be to functionalise the surface of the solid support, making it suitable for attachment of the molecules/polynucleotides. For example, silanes are known functional groups that have been used to attach molecules to a solid support material, usually a glass slide. The relatively short molecules and relatively long polynucleotides can then be brought into contact with the functionalised solid support, at suitable concentrations and in either separate or combined samples, to form the arrays.

In one preferred embodiment, the long polynucleotides and the short molecules each have the same reactive group that attaches to the solid support, or to an intermediary molecule.

In an alternative embodiment, the support surface may be treated with different functional groups, one of which is to react specifically with the relatively short molecules, and the other with the relatively long polynucleotides. Controlling the concentration of each functional group provides a convenient way to control the densities of the molecules/polynucleotides.

In a still further embodiment, the relatively short molecules are immobilised at high density onto the surface of the solid support. The molecules are capable of reacting with the polynucleotides (either directly or through an intermediate functional group) which can be brought into contact with the molecules at a suitable concentration to provide the required density. The polynucleotides are therefore immobilised on top of the monolayer of molecules. Those molecules that are not in contact with a polynucleotide may be reacted with a further molecule to block (or cap) the reactive site. This may be carried out before, during or after arraying the polynucleotides. The blocking (capping) group may itself be a relatively short polynucleotide.

Alternatively, only a minor proportion of the short molecules that are arrayed at high density on the solid support comprise a group that reacts with the polynucleotides; the majority are non-reactive. For example, the short molecules can be mixed silanes, a minor proportion ofwhich are reactive with a functional group on the polynucleotides, and the remaining silanes are unreactive and form the array of short molecules on the device. Therefore, controlling the concentration of the minor proportion of short molecules also controls the density of the polynucleotides.

In this embodiment, the short molecules may have been modified in solution prior to immobilisation on the array so that only a minor proportion contain a functional group that is capable of undergoing covalent attachment to a complementary functional group on the polynucleotides.

In a related embodiment, the short molecules are polynucleotides, and appropriate concentrations of both relatively long and relatively short polynucleotides are reacted with a functional group and then arrayed on the solid support, or to an intermediate molecule bound to the solid support.

Suitable functional groups will be apparent to the skilled person. For example, suitable groups include: amines, acids, esters, activated acids, acid halides, alcohols, thiols, disulfides, olefins, dienes, halogenated electrophiles and phosphorothioates. It is preferred if the group contains a silane.

The relatively small molecules may be any molecule that can provide a barrier against non-specific binding to the solid support.

Suitable small molecules may be selected based on the required properties of the surface and the existing functionality.

In a preferred embodiment, the molecules are silanes of type RₙSiX₍₄₋ₙ₎ (where R is an inert moiety that is displayed on the surface of the solid support and X is a reactive leaving group of type Cl or O-alkyl). The silanes include tetraethoxysilane, triethoxymethylsilane, diethoxydimethylsilane or glycidoxypropyltriethoxysilane, although many other suitable examples will be apparent to the skilled person.

In an embodiment of the invention, the short molecules act as surface blocks to prevent random polynucleotide association with the surface of the solid support. Molecules therefore require a group to react with the surface (which will preferably be the same functionality as used to attach the polynucleotide to the surface) and an inert group that will be defined by the properties required on the surface. In an embodiment, the surface is functionalised with an epoxide and the small molecule is glycine, although other compounds containing an amine group would suffice.

It is also preferred if the small molecule is hydrophilic and repels binding of anions. The molecule therefore may be acid, phosphate, sulfate, hydroxyl or polyol and may include polyethers such as PEG.

In one embodiment, the relatively short molecules are polynucleotides. These may be prepared using any suitable technique, including synthetic techniques known in the art. It may be preferable to use short polynucleotides that are immobilised to the solid support at one end and comprise, at the other end, a non-reactive group, e.g, a dideoxynucleotide incapable of incorporating further nucleotides. The short polynucleotide may also be a hairpin construct, provided that it does not interact with a polymerase.

In one embodiment of the present invention, each relatively long polynucleotide of the array comprises a hairpin loop structure, one end of which comprises a target polynucleotide, the other end comprising a relatively short polynucleotide capable of acting as a primer in a polymerase reaction. This ensures that the primer is able to perform its priming function during a polymerase-based sequencing procedure, and is not removed during any washing step in the procedure. The target polynucleotide is capable of being interrogated.

The term "hairpin loop structure" refers to a molecular stem and loop structure formed from the hybridisation of complementary polynucleotides that are covalently linked. The stem comprises the hybridised polynucleotides and the loop is the region that covalently links the two complementary polynucleotides. Anything from a 5 to 25 (or more) base pair double-stranded (duplex) region may be used to form the stem. In one embodiment, the structure may be formed from a single-stranded polynucleotide having complementary regions. The loop in this embodiment may be anything from 2 or more non-hybridised nucleotides. In a second embodiment, the structure is formed from two separate polynucleotides with complementary regions, the two polynucleotides being linked (and the loop being at least partially formed) by a linker moiety. The linker moiety forms a covalent attachment between the ends ofthe two polynucleotides. Linker moieties suitable for use in this embodiment will be apparent to the skilled person. For example, the linker moiety may be polyethylene glycol (PEG).

If the short molecules are polynucleotides in a hairpin construct, it is possible to ligate the relatively long polynucleotides to a minor proportion ofthe hairpins either prior to or after arraying the hairpins on the solid support.

The arrays have many applications in methods which rely on the detection of biological or chemical interactions with polynucleotides. For example, the arrays may be used to determine the properties or identities of cognate molecules. Typically, interaction of biological or chemical molecules with the arrays are carried out in solution.

In particular, the arrays may be used in conventional assays which rely on the detection of fluorescent labels to obtain information on the arrayed polynucleotides. The arrays are particularly suitable for use in multi-step assays where the loss of synchronisation in the steps was previously regarded as a limitation to the use of arrays. The arrays may be used in conventional techniques for obtaining genetic sequence information. Many of these techniques rely on the stepwise identification of suitably labelled nucleotides, referred to in US-A-5634413 as "single base" sequencing methods.

In an embodiment of the invention, the sequence of a target polynucleotide is determined in a similar manner to that described in US-A-5634413, by detecting the incorporation of nucleotides into the nascent strand through the detection of a fluorescent label attached to the incorporated nucleotide. The target polynucleotide is primed with a suitable primer (or prepared as a hairpin construct which will contain the primer as part of the hairpin), and the nascent chain is extended in a stepwise manner by the polymerase reaction. Each of the different nucleotides (A, T, G and C) incorporates a unique fluorophore at the 3' position which acts as a blocking group to prevent uncontrolled polymerisation. The polymerase enzyme incorporates a nucleotide into the nascent chain complementary to the target, and the blocking group prevents further incorporation of nucleotides. The array surface is then cleared of unincorporated nucleotides and each incorporated nucleotide is "read" optically by a charge-coupled device using laser excitation and filters. The 3' -blocking group is then removed (deprotected), to expose the nascent chain for further nucleotide incorporation.

Because the array consists of distinct optically resolvable polynucleotides, each target polynucleotide will generate a series of distinct signals as the fluorescent events are detected. Details of the full sequence are then determined.

Other suitable sequencing procedures will be apparent to the skilled person. In particular, the sequencing method may rely on the degradation of the arrayed polynucleotides, the degradation products being characterised to determine the sequence.

An example of a suitable degradation technique is disclosed in WO-A- 95/20053, whereby bases on a polynucleotide are removed sequentially, a predetermined number at a time, through the use of labelled adaptors specific for the bases, and a defined exonuclease cleavage.

A consequence of sequencing using non-destructive methods is that it is possible to form a spatially addressable array for further characterisation studies, and therefore non-destructive sequencing may be preferred. In this context, the term "spatially addressable" is used herein to describe how different molecules may be identified on the basis of their position on an array.

Once sequenced, the spatially addressed arrays may be used in a variety of procedures which require the characterisation of individual molecules from heterogeneous populations.

The following Examples illustrate the invention, with reference to the accompanying drawings.

### Example 1

Glass slides were cleaned with decon 90 for 12 h at room temperature prior to use, rinsed with water, EtOH and dried. A solution of glycidoxypropyltrimethoxysilane (0.5 mL) and mercaptopropyltrimethoxysilane (0.0005 mL) in acidified 95% EtOH (50 mL) was mixed for 5 min. The clean, dried slides were added to this mixture and left for 1 h at room temperature rinsed with EtOH, dried and cured for 1 h at 100°C. Maleimide modified DNA was prepared from a solution of amino-DNA (5'-Cy3-CtgCTgAAgCgTCggCAggT-heg-aminodT-heg-ACCTgCCgACgCT; SEQ ID NO.1) (10 µM, 100 µL) and N-[γ-Maleimidobutryloxy]succinimide ester (GMBS); (Pierce) (1 mM) in DMF/diisopropylethylamine (DIPEA)/water (89/1/10) for 1 h at room temperature. The excess cross-linker was removed using a size exclusion cartridge (NAP5) and the eluted DNA freeze-dried in aliquots and freshly diluted prior to use. An aliquot of the maleimide-GMBS-DNA (100 nM) was placed on the thiol surface in 50 mM potassium phosphate/l mM EDTA (pH 7.6) and left for 12 h at room temperature prior to washing with the same buffer.

The slide was inverted so that the chamber coverslip contacted the objective lens of an inverted microscope (Nikon TE200) via an immersion oil interface. A 60° fused silica dispersion prism was optically coupled to the back of the slide through a thin film of glycerol. Laser light was directed at the prism such that at the glass/sample interface it subtended an angle of approximately 68° to the normal of the slide and subsequently underwent Total Internal Reflection (TIR). Fluorescence from the surface produced by excitation with the surface specific evanescent wave generated by TIR was collected by the objective lens of the microscope and imaged onto an intensified charged coupled device (ICCD) camera (Pentamax, Princeton Instruments).

Images were recorded using a combination of a 532 Nd:YAG laser with a 580DF30 emission filter (Omega optics), with an exposure of 500 ms and maximum camera gain and a laser power of 50 mW at the prism.

The presence of glycidoxypropyltrimethoxysilane gave improved results (Fig. 1a) compared to a control carried out in the absence of glycidoxypropyltrimethoxysilane.

### Example 2

Slides were cleaned with decon 90 for 12 h prior to use and rinsed with water, EtOH and dried. A solution of tetraethoxysilane (0.7 mL) and N-(3-triethoxysilylpropyl)bromoacetamide (0.0007 mL) in acidified 95% EtOH (35 mL) was mixed for 5 min. The clean, dried slides were added to this mixture and left for 1 h at room temperature, rinsed with EtOH, dried and cured for 1 h at 100°C. Phosphorothioate modified DNA (5'-TMR-TACCgTCgACgTCgACgCTggCgAgCgTgCTgCggTTsTsTsTsT ACCgCAgCACgCTCgCCAgCg; SEQ ID NO. 2) where s = phosphorothioate (100 pM, 100 µL) in sodium acetate (30 mM, pH 4.5) was added to the surface and left for 1 h at room temperature. The slide was washed with a buffer containing 50 mM Tris/1 mM EDTA.

Imaging was performed as described in Example 1 and a good dispersion of single molecules was seen (Fig. 1b).

### Example 3

Slides were cleaned with decon 90 for 12 h prior to use and rinsed with water, EtOH and dried. A solution of glycidoxypropyltrimethoxysilane (0.5 mL) in acidified 95% EtOH was prepared and the cleaned slides placed in the solution for 1 h, rinsed with EtOH and dried. Amino modified DNA (5'-Cy3-CTgCTgAAgCgTCggCAggT-heg-aminodT-heg-ACCTgCCgACgCT; SEQ ID NO. 1) (1 µM, 100 µL) was placed on the surface and left for 12 h at room temperature. The slide was washed with a solution of 1 mM glycine at pH 9 for 1 h and flushed with 50 mM potassium phosphate/I mM EDTA (pH 7.6). A good dispersion of coupled single molecules was seen by TIR microscopy, as described in Example 1.

The slide was then exposed to a mixture containing Cy5-dUTP (20 µM) and T4 exo-polymerase (250 nM) and Tris (40 mM), NaCl (10 mM), MgCl₂ (4 mM), DTT (2 mM), potassium phosphate (1 mM), BSA (0.2 mgs/ml) 100 µL) at room temperature for 10 min. and then flushed with Tris/EDTA buffer.

Imaging was performed using a pumped dye laser at 630 nm with a 670DF40 emission filter at 40 mW laser power using the TIR setup as described. A lower level of non-specific trisphosphate binding was seen in the case using glycine, than in a control not treated with glycine.

## Claims

1. A device comprising a high density array of relatively short molecules and relatively long polynucleotides immobilised on the surface of a solid support, wherein each polynucleotide is immobilised by covalent bonding to the surface and adjacent polynucleotides of the array are separated by a distance of at least 10nm, whereby those parts of the polynucleotides that extend beyond the relatively short molecules can be individually resolved by optical means, and wherein less than 50%.of the long polynucleotides on the array are the same.

2. A device according to claim 1 wherein less than 30% of the polynucleotides on the array are the same.

3. A device according to claim 1 or claim 2 wherein the polynucleotides on the array are all different.

4. A device according to any one of claims 1 to 3, wherein the polynucleotides and the short molecules contain the same reactive group that attaches to the solid support.

5. A device according to any preceding claim wherein the polynucleotides are immobilised to the solid support via covalent attachment to an intermediate molecule and the short molecules are incapable of undergoing the same covalent attachment to the polynucleotides.

6. A device according to claim 5, wherein the intermediate molecule and the short molecules are silane compounds.

7. A device according to any preceding claim, wherein adjacent polynucleotides of the array are separated by a distance of at least 100nm.

8. A device according to any preceding claim, wherein the polynucleotides are separated by a distance of at least 250nm.

9. A device according to any preceding claim, wherein the device has a density of from 10⁶ to 10⁹ polynucleotides per cm².

10. A device according to any preceding claim, wherein the device has a density of from 10⁷ to 10⁸ polynucleotides per cm².

11. A device according to any preceding claim wherein the relatively short molecules are polynucleotides.

12. A device according to any preceding claim wherein each relatively long polynucleotide of the array comprises a hairpin loop structure.

13. Use of a device according to any preceding claim, for monitoring an interaction with a single polynucleotide, comprising resolving an arrayed polynucleotide with an imaging device.

14. A method of sequencing a target polynucleotide in a device according to any one of claims 1 to 12, wherein the sequence of the target polynucleotide is determined by detecting the incorporation of nucleotides into a nascent strand complementary to the target polynucleotide through the detection of a fluorescent label attached to the incorporated nucleotide.

15. A method according to claim 14 wherein each of the different nucleotides A, T, G and C incorporates a unique fluorophore at the 3' position which acts as a blocking group.

16. A method for the production of an array of polynucleotides which are at a density that permits individual resolution by optical means, comprising arraying on the surface of a solid support, a mixture of relatively long polynucleotides and relatively short molecules, wherein the short molecules are in excess of the polynucleotides, and wherein the polynucleotides and short molecules are brought into contact with the solid support in a single composition, the array being that described in any of claims 1-12.

17. A method according to claim 16 wherein the polynucleotides and the short molecules each have the same reactive group that attaches to the solid support or to an intermediate molecule.

18. A method according to claim 16 or claim 17 wherein the relatively short molecules are polynucleotides.

## Patentansprüche

1. Vorrichtung, umfassend eine Anordnung von relativ kurzen Moleküle und relativ langen Polynukleotide mit hoher Dichte, die auf der Oberfläche eines Festträgers immobilisiert sind, wobei jedes Polynukleotid durch eine kovalente Bindung an die Oberfläche immobilisiert ist und benachbarte Polynukleotide der Anordnung mit einem Abstand von wenigstens 10 nm getrennt sind, wodurch solche Teile der Polynukleotide, die sich über die relativ kurzen Moleküle erstrecken, mit optischen Mitteln einzeln aufgelöst werden können und wobei weniger als 50 % der langen Polynukleotide auf der Anordnung gleich sind.

2. Vorrichtung nach Anspruch 1, wobei weniger als 30 % der Polynukleotide auf der Anordnung gleich sind.

3. Vorrichtung nach Anspruch 1 oder Anspruch 2, wobei die Polynukleotide auf der Anordnung alle unterschiedlich sind.

4. Vorrichtung nach einem der Ansprüche 1 bis 3, wobei die Polynukleotide und die kurzen Moleküle dieselbe reaktive Gruppe enthalten, die an den Festträger bindet.

5. Vorrichtung nach einem der vorhergehenden Ansprüche, wobei die Polynukleotide auf dem Festträger über eine kovalente Verbindung an ein Zwischenmolekül immobilisiert sind und die kurzen Moleküle nicht fähig sind, dieselbe kovalente Verbindung mit den Polynukleotiden einzugehen.

6. Vorrichtung nach Anspruch 5, wobei das Zwischenmolekül und die kurzen Moleküle Silan-Verbindungen sind.

7. Vorrichtung nach einem der vorhergehenden Ansprüche, wobei die benachbarten Polynukleotide der Anordnung mit einem Abstand von wenigstens 100 nm getrennt sind.

8. Vorrichtung nach einem der vorhergehenden Ansprüche, wobei die Polynukleotide mit einem Abstand von wenigstens 250 nm getrennt sind.

9. Vorrichtung nach einem der vorhergehenden Ansprüche, wobei die Vorrichtung eine Dichte von 10⁶ bis 10⁹ Polynukleotide pro cm² besitzt.

10. Vorrichtung nach einem der vorhergehenden Ansprüche, wobei die Vorrichtung eine Dichte von 10⁷ bis 10⁸ Polynukleotide pro cm² besitzt.

11. Vorrichtung nach einem der vorhergehenden Ansprüche, wobei die relativ kurzen Moleküle Polynukleotide sind.

12. Vorrichtung nach einem der vorhergehenden Ansprüche, wobei jedes der relativ langen Polynukleotide der Anordnung eine Haarnadel-Struktur umfasst.

13. Verwendung einer Vorrichtung nach einem der vorhergehenden Ansprüche zum Verfolgen einer Interaktion mit einem einzelnen Polynukleotid, umfassend das Auflösen eines angeordneten Polynukleotids mit einer Abbildungsvorrichtung.

14. Verfahren zur Sequenzierung eines Ziel-Polynukleotids in einer Vorrichtung nach einem der Ansprüche 1 bis 12, wobei die Sequenz des Ziel-Polynukleotids bestimmt wird, indem der Einbau der Nukleotide in einem naszierenden Strang, der komplementär zu dem Ziel-Polynukleotid ist, durch Detection einer an das eingebaute Polynukleotid gekoppelten Fluoreszenzmarkierung nachgewiesen wird.

15. Verfahren nach Anspruch 14, wobei jedes der unterschiedlichen Nukleotide A, T, G und C eine einzigartige Fluorophore an der 3'-Position einbaut, welche als Blockierungsgruppe wirkt.

16. Verfahren zur Herstellung einer Anordnung von Polynukleotiden mit einer Dichte, welche eine individuelle Auflösung durch optische Mittel ermöglicht, umfassend das Anordnen eines Gemisches von relativ langen Polynukleotiden und relativ kurzen Molekülen auf der Oberfläche eines Festträgers, wobei die kurzen Moleküle gegenüber den Polynukleotiden im Überschuss sind und wobei die Polynukleotide und kurzen Moleküle mit dem Festträger in einer einzelnen Zusammensetzung in Kontakt gebracht werden und die Anordnung einer der in den Ansprüchen 1 bis 12 beschriebenen Anordnung entspricht.

17. Verfahren nach Anspruch 16, wobei die Polynukleotide und die kurzen Moleküle jeweils dieselbe reaktive Gruppe besitzen, die an den Festträger oder an ein Zwischenmolekül bindet.

18. Verfahren nach Anspruch 16 oder Anspruch 17, wobei die relativ kurzen Moleküle Polynukleotide sind.

## Revendications

1. Dispositif comprenant un réseau à haute densité de molécules relativement courtes et de polynucléotides relativement longs immobilisés sur la surface d'un support solide, dans lequel chaque polynucléotide est immobilisé par liaison covalente à la surface et les polynucléotides adjacents du réseau sont séparés par une distance d'au moins 10 nm, moyennant quoi les parties des polynucléotides qui se prolongent au-delà des molécules relativement courtes peuvent être résolues individuellement par un moyen optique, et dans lequel moins de 50 % des polynucléotides longs sur le réseau sont identiques.

2. Dispositif selon la revendication 1, dans lequel moins de 30 % des polynucléotides sur le réseau sont identiques.

3. Dispositif selon la revendication 1 ou la revendication 2, dans lequel les polynucléotides sur le réseau sont tous différents.

4. Dispositif selon l'une quelconque des revendications 1 à 3, dans lequel les polynucléotides et les molécules courtes contiennent le même groupe réactif qui s'attache au support solide.

5. Dispositif selon l'une quelconque des revendications précédentes, dans lequel les polynucléotides sont immobilisés au support solide par l'intermédiaire d'un attachement covalent à une molécule intermédiaire et les molécules courtes sont incapables de subir le même attachement covalent aux polynucléotides.

6. Dispositif selon la revendication 5, dans lequel la molécule intermédiaire et les molécules courtes sont des composés de silane.

7. Dispositif selon l'une quelconque des revendications précédentes, dans lequel les polynucléotides adjacents du réseau sont séparés par une distance d'au moins 100 nm.

8. Dispositif selon l'une quelconque des revendications précédentes, dans lequel les polynucléotides sont séparés par une distance d'au moins 250 nm.

9. Dispositif selon l'une quelconque des revendications précédentes, ledit dispositif ayant une densité comprise entre 10⁶ et 10⁹ polynucléotides par cm².

10. Dispositif selon l'une quelconque des revendications précédentes, ledit dispositif ayant une densité comprise entre 10⁷ et 10⁸ polynucléotides par cm².

11. Dispositif selon l'une quelconque des revendications précédentes, dans lequel les molécules relativement courtes sont des polynucléotides.

12. Dispositif selon l'une quelconque des revendications précédentes, dans lequel chaque polynucléotide relativement long du réseau comprend une structure en épingle à cheveux.

13. Utilisation d'un dispositif selon l'une quelconque des revendications précédentes, pour contrôler une interaction avec un seul polynucléotide, comprenant la résolution d'un polynucléotide disposé en réseau avec un dispositif d'imagerie.

14. Procédé de séquençage d'un polynucléotide cible dans un dispositif selon l'une quelconque des revendications 1 à 12, dans lequel la séquence du polynucléotide cible est déterminée par détection de l'incorporation de nucléotides dans un brin naissant complémentaire du polynucléotide cible à travers la détection d'un marqueur fluorescent attaché au nucléotide incorporé.

15. Procédé selon la revendication 14, dans lequel chacun des différents nucléotides A, T, G et C incorpore un fluorophore unique au niveau de la position 3' qui agit comme un groupe bloquant.

16. Procédé pour la production d'un réseau de polynucléotides qui se trouvent à une densité qui permet une résolution individuelle par un moyen optique, comprenant une disposition en réseau, sur la surface d'un support solide, d'un mélange de polynucléotides relativement longs et de molécules relativement courtes, dans lequel les molécules courtes sont en excès par rapport aux polynucléotides, et dans lequel les polynucléotides et les molécules courtes sont amenés au contact du support solide dans une composition unique, le réseau étant celui qui est décrit selon l'une quelconque des revendications 1 à 12.

17. Procédé selon la revendication 16, dans lequel les polynucléotides et les molécules courtes possèdent chacun le même groupe réactif qui s'attache au support solide ou à une molécule intermédiaire.

18. Procédé selon la revendication 16 ou la revendication 17, dans lequel les molécules relativement courtes sont des polynucléotides.
